# EUROPEAN PATENT SPECIFICATION

(11) **EP 3 829 455 B1**
(45) Date of publication and mention of the grant of the patent: **17.07.2024**
(21) Application number: 19844413.5
(22) Date of filing: 01.08.2019
(51) Int. Cl.: A61B 17/064, A61B 17/08, A61B 17/122, A61L 31/02, A61L 31/14

(54) **SIDEWAYS OR TANGENTIALLY APPLICABLE SURGICAL CLIP FOR BLEEDING CONTROL**
SEITLICH ODER TANGENTIAL ANWENDBARE CHIRURGISCHE KLAMMER ZUR BLUTUNGSKONTROLLE
CLIP CHIRURGICAL APPLICABLE LATÉRALEMENT OU TANGENTIELLEMENT POUR UNE COMMANDE DE SAIGNEMENT

(30) Priority: 02.08.2018 US 201862713704 P
(43) Date of publication of application: 09.06.2021
(73) Proprietor: Siegenthaler, Michael, Potomac, Maryland 20854 (US)
(72) Inventor: Siegenthaler, Michael, Potomac, Maryland 20854 (US)
(74) Representative: Tostmann, Holger Carl
(86) International application number: PCT/US2019/044641
(87) International publication number: WO 2020/028649

(56) References cited:
- EP-B1- 0 287 275
- WO-A1-98/18389
- US-A- 4 590 937
- US-A1- 2002 007 184
- US-A1- 2005 055 027
- US-A1- 2007 276 417
- US-A1- 2011 270 285
- US-A1- 2016 199 113
- US-B1- 6 226 843
- US-B1- 6 425 903

## Description

### BACKGROUND

### FIELD

The present disclosure is directed to surgical clips that prevent or control bleeding during surgical procedures. More specifically, the present disclosure is directed to novel surgical clips that may be applied tangentially, or sideways, in order to prevent or control bleeding.

### DESCRIPTION OF THE RELATED ART

Surgical clips are implantable medical devices, applied by a clip applicator, that have been in use since the first half of the twentieth century. Today surgical clips are widely used by surgeons of multiple disciplines during conventional open and minimally invasive surgical procedures. Surgical clips are deployed using a clip applicator which holds clip in an open position. Once the clip is positioned over a blood vessel, the clip applicator, which has scissor-like handles and plier-like tips, can be squeezed to deform the surgical clip into its final form. Once deformed, the surgical clip occludes the blood vessel, closing its lumen. After installation of the surgical clip, the blood vessel may be divided, or cut, without causing excessive bleeding. For typical blood vessel division, surgical clips are placed at either side of the division point before the vessel is divided. Blood vessels for which surgical clips are used are fairly small, typically no more than a few millimeters in maximal diameter.

Conventional surgical clips are flat, and typically made of metal, such as stainless steel, titanium, talanum, platinum, or metal alloys, and clips made of biodegradable polymers are also available. Their flat design has the disadvantage that if a small side branch of a larger blood vessel is injured at the point where the small side branch meets the larger blood vessel, a conventional surgical clip cannot be used to control bleeding. This is because the lager blood vessel may be too large to be controlled with a clip, and may be anatomically too important to be permanently occluded. In situations where there is an injury at a location where a small blood vessel meets a larger blood vessel, instead of using conventional surgical clips, a surgeon must resort to time-consuming sutures and suture knots. These sutures and suture knots can be very technically difficult, and may not be safely possible due to size constraints during, for example, minimally invasive procedures. Under these circumstances the procedure may have to become more invasive through the use of a large incision, which often makes patient recovery more difficult. Since patient outcome of surgical procedures is related to the time of the procedure, and shorter operative times are advantageous for patients, such large incision, open procedures are to be avoided, if possible without compromising patient safety.

Another problem with conventional surgical clips is their limited applicability to venous injuries. Bleeding in venous injuries can be very difficult to control because the walls of larger veins can be very thin and tend to tear when surgical sutures are placed to repair the injury. Thus, repair attempts on injured veins can make the bleeding worse and can even lead to patient death.

Vascular injuries at locations where a small blood vessel meets a larger blood vessel are common since they may occur due to traction on the smaller blood vessel, which leads to an avulsion injury of the small blood vessel where it originates from the larger blood vessel. Venous injuries where small side branches meet a large vein also occur commonly. Thus, there is a need for a surgical clip that can be used in these situations where conventional surgical clips cannot be used, and where sutures and suture nots may be difficult and even dangerous to use.

US 4 590 937 relates to a nonmetallic surgical clip having two separate arms joined together at an apex is disclosed. The clip comprises a weakened portion and a bend in at least one arm. The bend is at the weakened portion. The amount of the bend is such that at least a part of one arm is in an essentially parallel relationship with at least a part of the other arm.

EP 287 275 relates to a hemostatic clip for surgical use is disclosed having grooves on the inner surfaces of the legs which form an asterisk type pattern upon closure, knee bends in each leg which are opposed by notches or grooves that enhance the ability of the legs to fully straighten upon closure, and distal flat surfaces on the ends of the clip legs to prevent scissoring and ensure hemostasis when the clip is closed.

WO 98/18389 relates to hemostatic clips that are used when control of a vessel has not been obtained or when a vessel is lacerated or divided inadvertently. These clips urge closure of a perforation in the wall of a vessel and reduce the necessity for suturing the vessel. In addition, the disclosure addresses the need for a hemostatic clip that can be used to efficiently ligate a severed vessel.

US 6 425 903 relates to an implantable marker for implantation in tissue of a surgical patient is disclosed. The marker has a base and an elevated bridge. A pair of legs descend from first and second transitions of the bridge. Each leg has a distal tip, a generally straight leg arm adjacent the tip, a camming marker surface between the transitions of the base and the straight leg arm, and a camming marker surface notch. The camming marker surfaces extend outwardly from the straight leg arms of the legs.

### SUMMARY

The present disclosure is directed to a novel surgical clip that is curved such that vascular and venous injuries, such as those described above, can be repaired safely and quickly in order to limit blood loss.

As such, in one aspect of the present disclosure a surgical clip that includes two leg sections arranged substantially parallel to each other, two straight sections respectively attached to the two leg sections at an angle to the two leg sections, and at least one curved section connecting the two straight sections at ends opposite to the two leg sections.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
Fig. 1a illustrates a plurality of conventional surgical clips and their respective applicators;
Fig. 1b illustrates three conventions surgical clips in various stages from open to fully closed;
Fig. 2a is a first view of a surgical clip according to exemplary aspects of the present disclosure;
Fig. 2b is a second view of a surgical clip according to exemplary aspects of the present disclosure;
Fig. 2c is a third view of a surgical clip according to exemplary aspects of the present disclosure;
Fig. 2d is a fourth view of a surgical clip according to exemplary aspects of the present disclosure;
Fig. 2e is a fifth view of a surgical clip according to exemplary aspects of the present disclosure;
Fig. 3a is a first view of another surgical clip according to exemplary aspects of the present disclosure;
Fig. 3b is a second view of the other surgical clip according to exemplary aspects of the present disclosure;
Fig. 4a is a first view of a further surgical clip according to exemplary aspects of the present disclosure;
Fig. 4b is a second view of the further surgical clip according to exemplary aspects of the present disclosure;
Fig. 4c is a third view of the further surgical clip according to exemplary aspects of the present disclosure;
Fig. 5a is a first view of another surgical clip according to exemplary aspects of the present disclosure;
Fig. 5b is a second view of the other surgical clip according to exemplary aspects of the present disclosure;
Fig. 6a is an illustration of a traction injury causing bleeding at the junction of a small side branch to a large blood vessel;
Fig. 6b is an illustration of a blood vessel injury from a sharp surgical instrument;
Fig. 6c is a first step in the application of a surgical clip which is now positioned in it's open configuration an accurate position to control the bleeding site according to exemplary aspects of the present disclosure;
Fig. 6d is a second step in the application of a surgical clip, which is now being closed according to exemplary aspects of the present disclosure;
Fig. 6e is a third step in the application of a surgical clip with the clip closed and the bleeding controlled according to exemplary aspects of the present disclosure;
Fig. 6f is a final result of the application of a surgical clip controlling the large blood vessel according to exemplary aspects of the present disclosure;
Fig. 7a is a surgical clip according to additional exemplary aspects of the present disclosure; and
Fig. 7b is another view of the surgical clip according to the additional aspects of the present disclosure.

### DETAILED DESCRIPTION

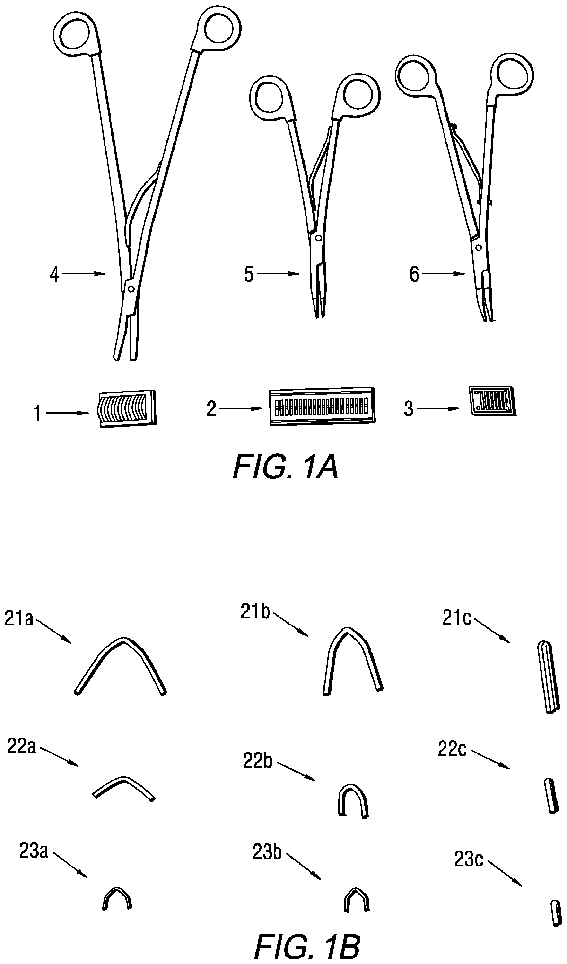

Referring now to the drawings, wherein like reference numerals designate identical or corresponding parts throughout the several views, Fig. 1a is view of conventional surgical clips and their respective applicators 4, 5, 6. As illustrated in Fig. 1a, the applicators 4, 5, 6 are scissor-like devices that are used to hold and deform a surgical clip into its final form in order to occlude a blood vessel onto which the surgical clip is applied. As noted above, the surgical clips may be made of metal, such as stainless steel, titanium, talanum, platinum, or metal alloys and clips made of polymers, which can be biodegradable, are also available. The surgical clips also come in stacks 1, 2, 3, as illustrated in this figure.

Fig. 1b shows surgical clips 21, 22, 23 of three different dimensions and in three states of deformation. The clip 21 at the top of Fig. 1b is the largest, and the clip 23 at the bottom is the smallest. From left to right, the clips are shown open, partially closed, and fully closed. As can be appreciated the clip sizes shown in Fig. 1b are merely exemplary, and both larger and smaller clips are possible without departing from the scope of the present disclosure.

In Fig. 1b, the surgical clips 21, 22, 23, regardless of size, all have a single bend roughly in their middle in order to form a V-like shape. Since this is the only bend in the clips of Fig. 1b, these clips 21, 22, 23 can lie flat on a flat surface. Thus, these conventional clips cannot be used in application where the injury to a blood vessel is very close to a point where the blood vessel meets a larger blood vessel, or in repair of venous injuries, as discussed above.

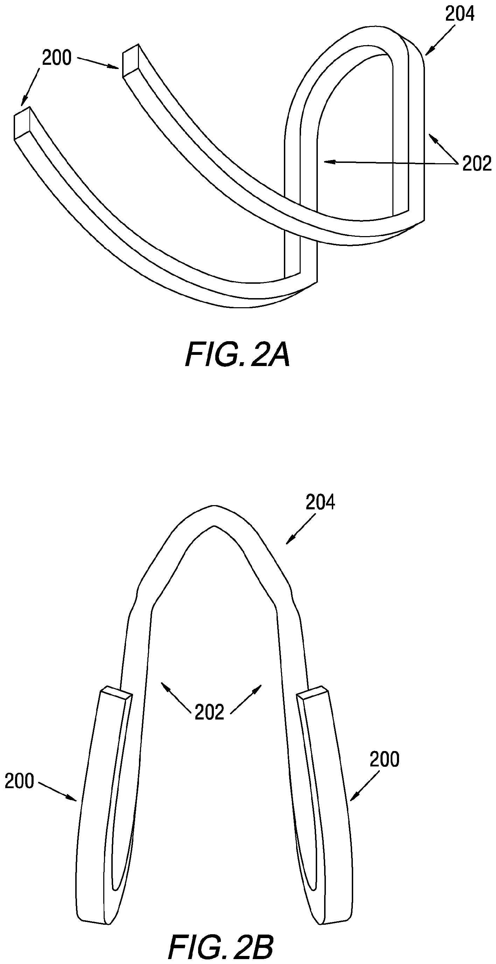

Fig. 2a is a first view of a surgical clip according to exemplary aspects of the present disclosure. The surgical clip in Fig. 2a includes two curved legs 200 that are connected to respective straight sections 202 that are joined together by a semi-circular curved section 204. Though the surgical clip in Fig. 2a is described in terms of different sections for clarity, one of ordinary skill would recognize that the surgical clip in Fig. 2a may be formed as one piece. Moreover, the semi-circular curved section 204 is illustrated as a semi-circle merely as an example, this section may also be bent in a "V" shape, a square shape, or any other shape that is known. The cross-section of the different sections of the clip in Fig. 2a is illustrated as being square or rectangular. However, this cross-section can also be circular, oblong, triangular, or any other shape, or combination of shapes, without departing from the scope of the present disclosure and can be a v-shape on both ends of the limbs

Fig. 2b is a second view of the surgical clip in Fig. 2a. In this view, the surgical clip is shown "head-on" illustrating that the curved section 204 can also be V shaped such that the straight sections 202 splay out by a predetermined amount. As can be seen from this figure, the curved legs 200 curve up to about half of the height of the surgical clip. However, this is exemplary as, depending on application, the curve in the legs 200 of the clip may be more or less that what is illustrated in Fig. 2b.

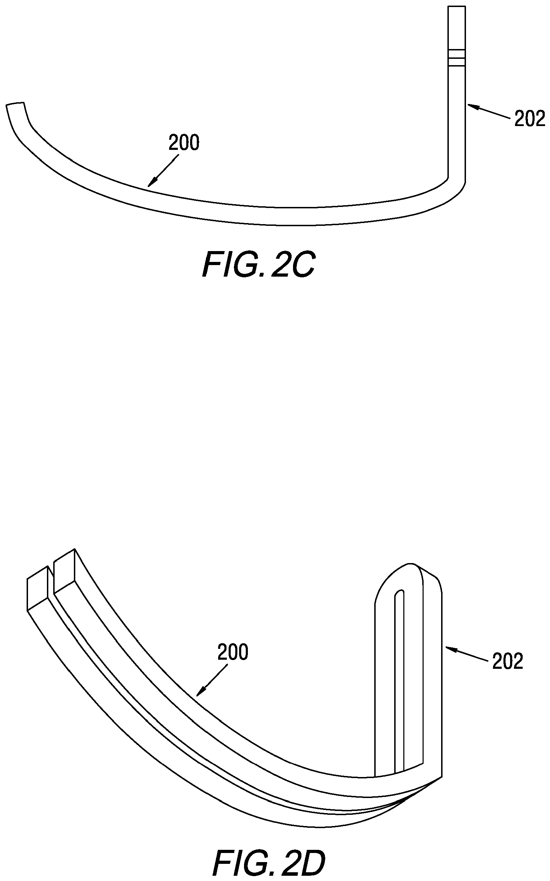

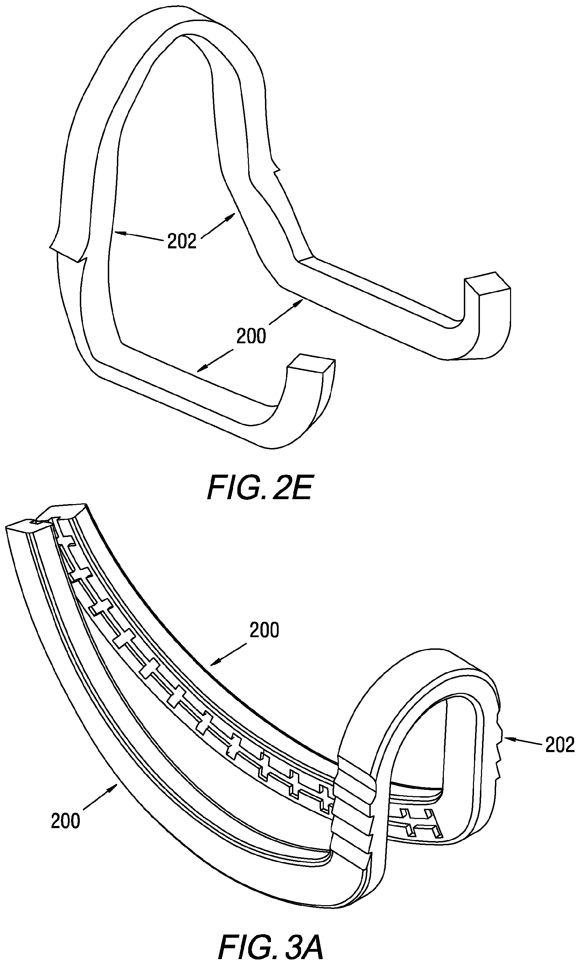

Fig. 2c is a side view of the surgical clip of Figs. 2a and 2b, and Fig. 2d shows the surgical clip in its closed state. The design of the surgical clip in Figs. 2a-2d advantageously allows the inclusion of larger blood vessels near the curved section 204 allowing this surgical clip to be applied to blood vessels at the point in which the blood vessel meets a larger blood vessel. Fig. 2e shows a variant of the surgical clip of Figs. 2a-2d. In Fig. 2e the straight sections 202 of the clip are reinforced to ensure a secure closing at the apposition zone. As can be appreciated, this clip may be made of metal, such as stainless steel, titanium, talanum, platinum, or metal alloys or polymers.

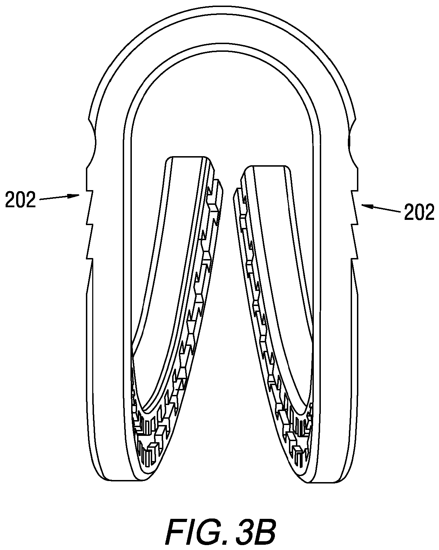

Fig. 3a is a first view of another surgical clip according to exemplary aspects of the present disclosure. The surgical clip illustrated in Fig. 3a may have substantially the same shape as the clip in Figs. 2a-2e. However, the apposition zone of this clip includes a profile, or texture, in order to prevent slipping once the clip is installed. As can be appreciated, the profile or texture illustrated in Fig. 3a is merely exemplary and other profile or texture patterns are possible without departing from the scope of the present disclosure. The straight sections 202 of the clip in Fig. 3a also include a texture to avoid slipping while being held in the applicator. Fig. 3b is a second view of the back of the surgical clip in Fig. 3a.

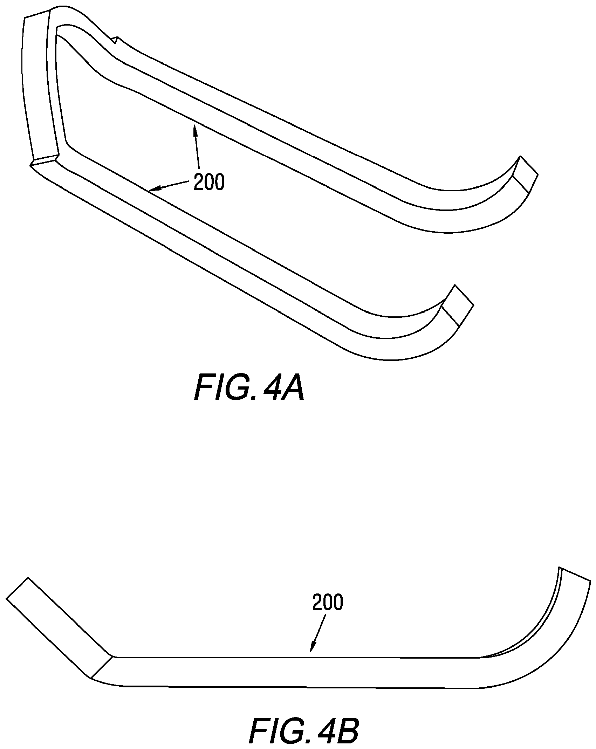

Fig. 4a illustrates a first view of a further surgical clip according to exemplary aspects of the present disclosure. The surgical clip in Fig. 4a is substantially the same as those described above, except that the curve in the legs 200 of the clip is shallower and may even have a portion that is straight. What is different in this example compared to the previous illustrations, is that the clip applicator is to be applied to the long portion of the clip, which allows this or a similar shallow configuration to be used for saphenous vein harvested conduits for coronary bypass surgery, where a tangential clip is required and minimal narrowing of the venous bypass conduit is of paramount importance.

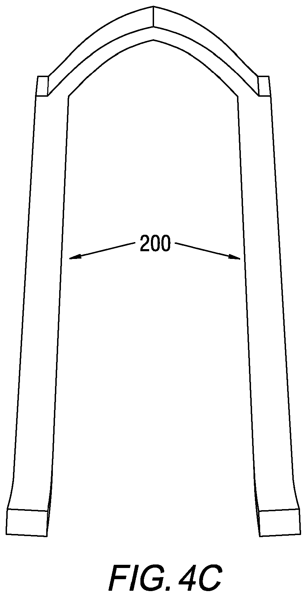

Fig. 4b is a side view of the clip in Fig. 4a, and Fig. 4c is a top view. As can be appreciated, the surgical clip in Figs. 4a-4c may be made as a one-piece clip, like the other clips described herein, or may be made in different sections that are joined by, for example, soldering. As can be seen in Fig 4c, the long leg 200 can be bulkier than the shallow v-shaped part that connects the 2 long limbs, this allows the clip to be loaded on a clip applicator by sliding the clip into corresponding grooves in the clip applicator brackets. The clip can also be made of metal, such as stainless steel, titanium, talanum, platinum, metal alloys or polymers, which can be bioabsorbable.

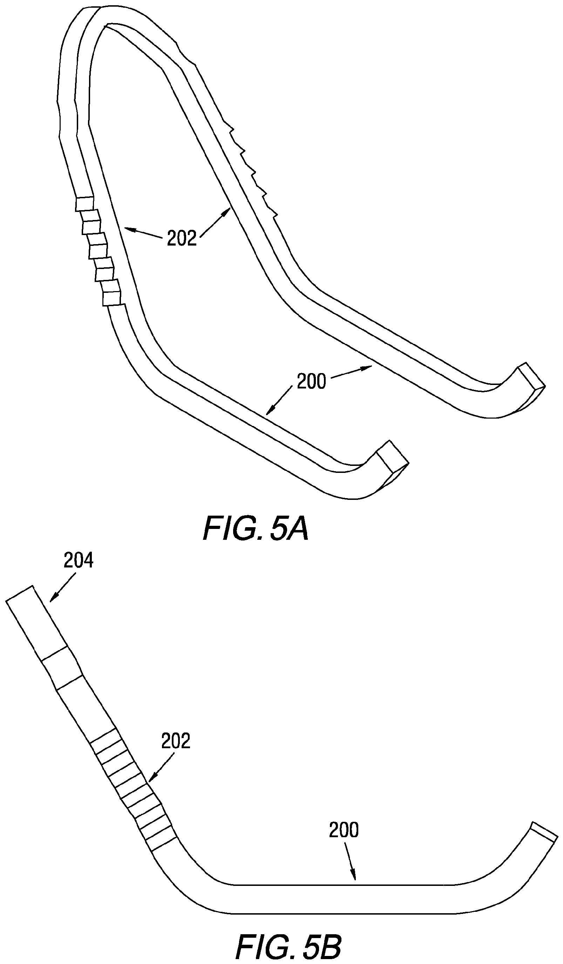

Fig. 5a is a first view of another surgical clip according to exemplary aspects of the present disclosure. Fig. 5b is a side view of the surgical clip in Fig. 5a. In this clip, the curved section 204 and the straight sections 202 are arranged at an oblique angle relative to the legs 200, allowing control of blood vessels running at different angles during certain minimally invasive procedures than the previous drawings in Figs 1. Otherwise, this clip may be formed of the same materials and in the same way as the other clips described herein.

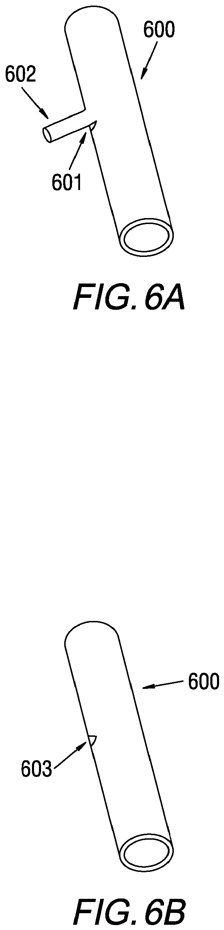

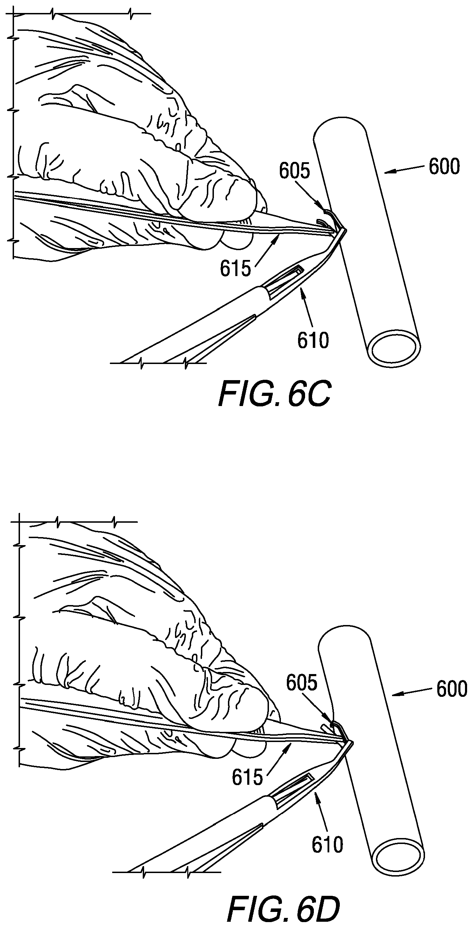

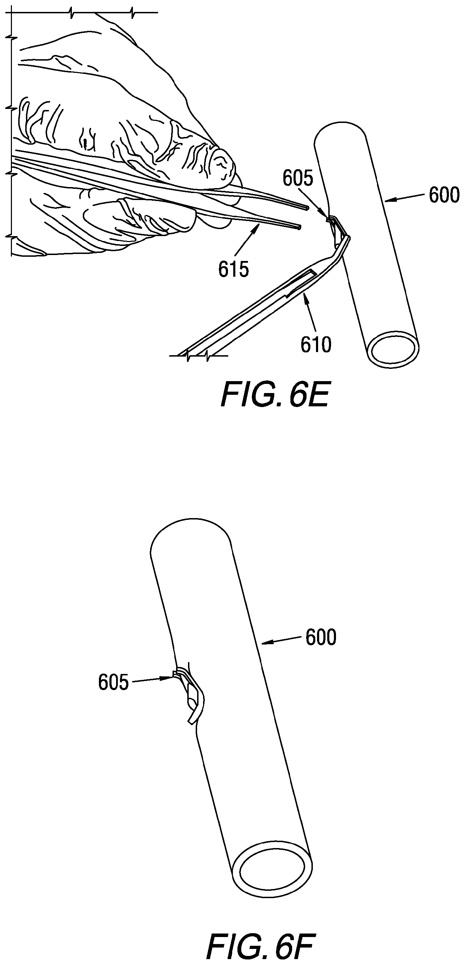

Fig.s 6a and 6b illustrate vascular injuries prior to applying a surgical clip according to exemplary aspects of the present disclosure. Fig 6a illustrates a traction injury 601 to a small side branch 602 of a blood vessel 600, and Fig. 6b illustrates a surgical injury 603 to the blood vessel 600 due to a sharp surgical instrument. In Fig. 6c a surgical clip 605 according to exemplary aspects of the present disclosure is applied to an anatomically important blood vessel 600 which can be an artery or a vein, with an injury. As can be seen in this figure, the applicator 610 of the surgical clip 605 holds the clip by the curved section thereof which allows placement of the straight sections of the clip 605 over the injury. The portion of the blood vessel 600 where the surgical clip 605 is to be applied is held with forceps 615. The clip applicator 610 is used to move the clip 605 into position while the blood vessel 600 is being held by the forceps 615. Subsequently, in Fig. 6d, pressure is applied to the handles of the applicator 610 in order to cause the surgical clip 605 to close around the injury of the blood vessel 600. In Fig. 6e, the forceps 615 may be withdrawn as further pressure is applied to the surgical clip 605 to ensure that the surgical clip 605 is fully closed. This maneuver allows the surgeon to inspect if the injury has been fully controlled prior to opening the clip applier and if needed, to grab the injured section again without losing control of the blood vessel 600 and place another clip, if needed. Fig. 6f shows the clip fully closed and installed at the injured portion of the blood vessel. As can be appreciated, the application technique illustrated in Figs. 6a-f is merely exemplary and other techniques are possible as one of ordinary skill would recognize.

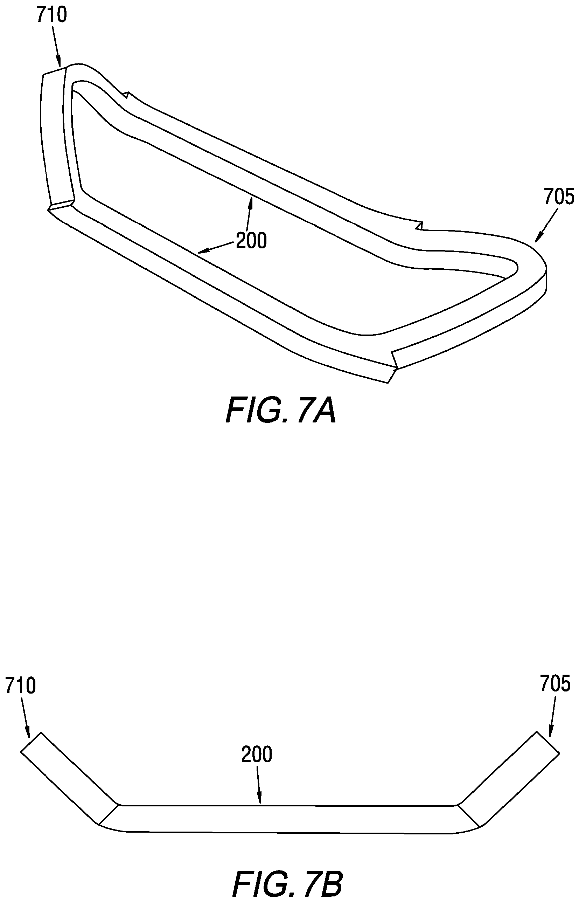

Figs. 7 a and 7b show an oblique and side view of another surgical clip with bulkier legs 200 similar to Fig. 4 where the clip applicator is inserted for clip placement and 2 v-shaped bends 705, 710 connecting each end of the bulkier limbs of the clip.

## Claims

1. A surgical clip (21, 22, 23), comprising:
two leg sections arranged substantially parallel to each other;
two straight sections (202) respectively attached to the two leg sections at an angle to the two leg sections; and
at least one curved section (200) connecting the two straight sections (202) at ends opposite to the two leg sections,
**characterized in**
**that** each of the two leg sections are curved (202), and that each of the two leg sections (200) have a straight portion.

2. The surgical clip (21, 22, 23) according to claim 1, wherein the curved section (200) is configured to bend under a compressive force in order to reduce a distance between the two leg sections.

3. The surgical clip (21,22, 23) according to claim 1, wherein each of the two leg sections include a flat portion.

4. The surgical clip (21, 22, 23) according to claim 1, wherein the surgical clip is formed from one piece of material.

5. The surgical clip (21, 22, 23) according to claim 4, wherein the material is one of stainless steel, titanium, talanum, platinum, and a metal alloy or a polymer, which can be reabsorbable.

6. The surgical clip according (21, 22, 23) to claim 1, wherein each of the leg sections include a textured surface configured to prevent slippage once the surgical clip is installed.

7. The surgical clip (21, 22, 23) according to claim 1, wherein each of the straight sections (202) include a textured surface to prevent slippage when held in an applicator device.

## Patentansprüche

1. Chirurgische Klammer (21, 22, 23), welche aufweist:
zwei im Wesentlichen parallel zueinander angeordnete Schenkelabschnitte;
zwei gerade Abschnitte (202), die jeweils an den beiden Schenkelabschnitten in einem Winkel zu den beiden Schenkelabschnitten angebracht sind; und
mindestens einen gebogenen Abschnitt (200), der die beiden geraden Ab-schnitte (202) an Enden, die den beiden Schenkelabschnitten gegenüberliegen verbindet, **dadurch gekennzeichnet**
**dass** jeder der beiden Schenkelabschnitte (202) gekrümmt ist und dass jeder der beiden Schenkelabschnitte (200) einen geraden Abschnitt aufweist.

2. Chirurgische Klammer (21, 22, 23) nach Anspruch 1, wobei der gekrümmte Abschnitt (200) ausgestaltet ist, um sich unter einer Druckkraft zu biegen, um einen Abstand zwischen den beiden Schenkelabschnitte zu verringern.

3. Chirurgische Klammer (21, 22, 23) nach Anspruch 1, wobei jeder der beiden Schenkelabschnitte einen flachen Abschnitt aufweist.

4. Chirurgische Klammer (21, 22, 23) nach Anspruch 1, wobei die chirurgische Klammer aus einem Stück eines Materials gebildet worden ist.

5. Chirurgische Klammer (21, 22, 23) nach Anspruch 4, wobei das Material eines der folgenden ist: rostfreier Stahl, Titan, Talan, Platin, eine Metalllegierung oder ein Polymer, das resorbierbar sein kann.

6. Chirurgische Klammer nach (21, 22, 23) Anspruch 1, wobei jeder der Schenkel-abschnitte eine strukturierte Oberfläche aufweist, die ausgestaltet ist, um ein Ver-rutschen zu verhindern, sobald die chirurgische Klammer angebracht worden ist.

7. Chirurgische Klammer (21, 22, 23) nach Anspruch 1, wobei jeder der geraden Ab-schnitte (202) eine texturierte Oberfläche aufweist, um ein Verrutschen zu verhindern, wenn diese in einer Applikatorvorrichtung gehalten wird.

## Revendications

1. Agrafe chirurgicale (21, 22, 23), comprenant :
deux sections de patte disposées de façon sensiblement parallèle l'une à l'autre ;
deux sections droites (202) respectivement fixées aux deux sections de patte selon un certain angle par rapport aux deux sections de patte ; et
au moins une section incurvée (200) reliant les deux sections droites (202) au niveau d'extrémités opposées aux deux sections de patte,
**caractérisée**
**en ce que** chacune des deux sections de patte est incurvée (202), et en ce que chacune des deux sections de patte (200) a une partie droite.

2. Agrafe chirurgicale (21, 22, 23) selon la revendication 1, dans laquelle la section incurvée (200) est configurée pour se plier sous l'effet d'une force de compression afin de réduire une distance entre les deux sections de patte.

3. Agrafe chirurgicale (21, 22, 23) selon la revendication 1, dans laquelle chacune des deux sections de patte comprend une partie plate.

4. Agrafe chirurgicale (21, 22, 23) selon la revendication 1, dans laquelle l'agrafe chirurgicale est formée d'une seule pièce de matériau.

5. Agrafe chirurgicale (21, 22, 23) selon la revendication 4, dans laquelle le matériau est l'un parmi acier inoxydable, titane, tantale, platine, et un alliage métallique ou un polymère, qui peut être résorbable.

6. Agrafe chirurgicale (21, 22, 23) selon la revendication 1, dans laquelle chacune des sections de patte comprend une surface texturée configurée pour empêcher le glissement une fois l'agrafe chirurgicale installée.

7. Agrafe chirurgicale (21, 22, 23) selon la revendication 1, dans laquelle chacune des sections droites (202) comprend une surface texturée pour empêcher le glissement lorsqu'elle est tenue dans un dispositif applicateur.
